# EUROPÄISCHE PATENTSCHRIFT

(11) **EP 1 572 620 B1**
(45) Veröffentlichungstag und Bekanntmachung des Hinweises auf die Patenterteilung: **23.10.2013**
(21) Anmeldenummer: 03767767.1
(22) Anmeldetag: 05.12.2003
(51) Int. Cl.: C07C 69/72, C07C 67/56, C07D 309/32

(54) **VERFAHREN ZUR HERSTELLUNG OPTISCH AKTIVER DIHYDROPYRONE**
METHOD FOR THE PRODUCTION OF OPTICALLY ACTIVE DIHYDROPYRONES
PROCEDE DE PREPARATION DE DIHYDROPYRONE OPTIQUEMENT ACTIVE

(30) Priorität: 10.12.2002 DE 10257761
(43) Veröffentlichungstag der Anmeldung: 14.09.2005
(73) Patentinhaber: Boehringer Ingelheim Pharma GmbH & Co. KG, 55216 Ingelheim am Rhein (DE)
(72) Erfinder: SAUTER, Markus, 55457 Gensingen (DE); SCHRÖDER, Jürgen, 55128 Mainz (DE); JÄGER, Burkhard, 55411 Bingen 13 (DE)
(86) Internationale Anmeldenummer: PCT/EP2003/013851
(87) Internationale Veröffentlichungsnummer: WO 2004/052831

(56) Entgegenhaltungen:
- WO-A-98/19997
- DE-C- 10 108 471
- TURNER S R ET AL: "Tipranavir(PNU-140690) A potent, Orally Bioavailable Nonpeptidic HIV protease inhibitor of the 5,6-Dihydro-4-hydroxy-2-pyrone Sulfonamide Class" JOURNAL OF MEDICINAL CHEMISTRY, AMERICAN CHEMICAL SOCIETY. WASHINGTON, US, vol. 41, no. 18, 1998, pages 3467-3476, XP002194391 ISSN: 0022-2623 cited in the application

## Beschreibung

Die vorliegende Erfindung betrifft ein Verfahren zur Herstellung optisch aktiver 5-Hydroxy-3'-ketoester, die zur Herstellung von optisch aktiven Dihydropyronen als Ausgangsverbindungen für die Herstellung pharmazeutisch wirksamer Verbindungen verwendet werden können.

### Hintergrund der Erfindung

Da sich Enantiomere hinsichtlich ihrer physikalischen Eigenschaften nur sehr wenig, aber in ihren physiologischen Wirkungen deutlich unterscheiden, ist es für bestimmte Anwendungen von grundsätzlicher Bedeutung, die enantiomeren Formen voneinander getrennt zu erhalten. Dies spielt insbesondere für den Pharmaziebereich eine Rolle. Das Hauptziel ist hierbei die Erzeugung möglichst enantiomerenreiner Verbindungen, da ein Enantiomer mit anderen chiralen Verbindungen in unterschiedliche, nicht vorhersehbare Wechselwirkungen treten kann. Die Natur hat hierfür spezielle chirale Katalysatoren, wie Enzyme, entwickelt, die mit hohem Wirkungsgrad enantioselektiv arbeiten. Demgegenüber wird in der präparativen Chemie versucht, stereoselektiv verlaufende Synthesen bereitzustellen, die mit hoher Ausbeute nur zu einem Isomeren hoher optischer Reinheit führen. Dies ist nur in Ausnahmefällen umsetzbar. Eine andere Möglichkeit stellen physikalische Trennmethoden dar, mit denen die erzeugten Enantiomeren getrennt werden können.

Eine häufig eingesetzte Art der Enantiomerentrennung ist die sogenannte Racematspaltung, d. h. die Zerlegung einer Mischung mit gleichen Anteilen beider Enantiomeren in die optisch aktiven Komponenten. Eines der gängigsten Verfahren zur Enantiomerentrennung ist die chemische Spaltung racemischer Gemische, wobei entweder eine racemische, in die Enantiomeren aufzutrennende Säure mit einer optisch aktiven Base oder eine optisch aktive Säure mit einer racemischen Base unter Salzbildung umgesetzt wird. Hierdurch werden Diastereomere gebildet, die durch unterschiedliche Löslichkeiten voneinander getrennt werden können.

So beschreibt beispielsweise die US 4 661 628 ein Verfahren zur Trennung racemischer Mischungen von α-Naphthylpropionsäuren durch Zugabe eines ß-Aminoalkohols, wodurch die gebildeten diastereomeren Amide mit fraktionierter Kristallisation getrennt und anschließend durch Hydrolyse wieder in die optisch aktiven Säuren umgewandelt werden können. Diese Trennung der Enantiomeren ist erforderlich, da nur eines der beiden Isomere antiphlogistische Wirkung zeigt. Es ist international unter dem Namen Naproxen bekannt.

Weiterhin werden bekannte physikalische Verfahren zur Racematspaltung eingesetzt. So werden in der Gaschromatographie, Dünnschichtchromatographie, HPLC, Flüssig-flüssig-Extraktion oder -Verteilung Wechselwirkungen, wie Wasserstoff-Brückenbindungen, Liganden-Austausch und die Bildung von Metall- oder Charge-Transfer-Komplexen, zur Enantiomerentrennung herangezogen. Zur Enantiomerentrennung mit chromatographischen Verfahren werden im allgemeinen optisch aktive Adsorbentien eingesetzt, wobei chirale stationäre oder mobile Phasen Verwendung finden. Ein bekanntes Beispiel stellt die Racematspaltung der Trögerschen Base an Lactose dar.

Die Enantiomerentrennung spielt insbesondere im pharmazeutischen Bereich eine wichtige Rolle, wo ganz spezielle Verbindungen von Interesse sind, d.h. nur diejenige der enantiomeren Formen, welche die gewünschte pharmakologische und toxikologische Wirkung aufweist. Beispielsweise stellen die Enantiomeren der 5-Hydroxy-3-ketoester bzw. die daraus erhältlichen 5,6-Dihydro-4-hydroxy-2-pyrone wichtige Strukturelemente bei einer Vielzahl pharmazeutisch wirksamer Verbindungen dar, wobei die Klasse der 5,6-Dihydro-4-hydroxy-2-pyron-sulfonamide von besonderer Bedeutung sind, da sie als nicht-peptidische HIV-Protease-Inhibitoren zum Einsatz kommen. Ein besonders wirksames Beispiel für einen potenten und oral bioverfügbaren HIV-Protease-Inhibitor dieser Substanzklasse ist die Verbindung Tipranavir (PNU-140690), welche die folgende Struktur aufweist:

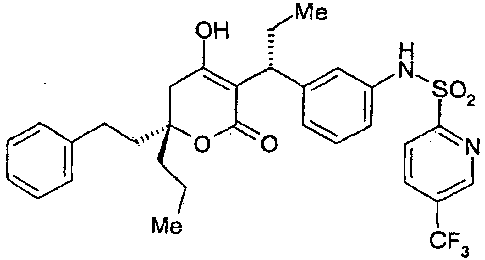

Diese wie auch andere strukturell ähnliche Verbindungen sind aus dem Stand der Technik bekannt (siehe beispielsweise J. Med. Chem. 1998, 41, 3467-3476).

Ein Schlüsselschritt bei der Synthese der vorstehend genannten, wie auch strukturell ähnlicher Verbindungen, ist die Umsetzung von 5,6-Dihydro-4-hydroxy-2-pyronen **1** mit geeignet substituierten Carbonylverbindungen **2** zu den Kondensationsprodukten **3**, wie dies im nachfolgenden Schema 1 dargestellt ist: 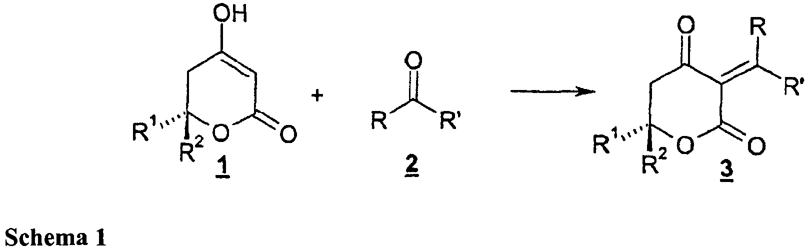 wobei die Bedeutung der voneinander verschiedenen Reste R¹ und R² der Beschreibung der vorliegenden Erfindung zu entnehmen ist. Die Reste R und R' sind variabel und bestimmen sich durch das Substitutionsmuster der jeweiligen Zielverbindungen, wie sie aus dem Stand der Technik hervorgehen.

Ein Weg zur Herstellung bzw. Gewinnung von Enantiomeren der optisch aktiven Dihydropyrone ist im Stand der Technik ebenfalls bereits bekannt. So beschreibt die WO 02/068404 A1 ein derartiges Verfahren, worin zunächst geeignet substituierte Carbonylverbindungen mit einem Acetessigsäurederivat in Gegenwart organischer oder anorganischer Basen zu einem racemischen Gemisch von 5,6-Dihydro-4-hydroxy-2-pyronen umgesetzt werden. Das erhaltene racemische Gemisch wird dann mit einem chiralen Aminoalkohol in die Salze überführt, wobei je nach Wahl des Aminoalkohols die gewünschten Salze der R- oder S-Konfiguration auskristallisiert werden können und das verbleibende Enantiomer in Lösung zurückbleibt.

In der WO 98/19997 werden Dihydropyrone mit verbesserter antiviraler Aktivität und deren Herstellung beschrieben. Das dort in Schema 8 dargestellte Verfahren sieht die Isolierung von 5-Hydroxy-3-ketoestern vor, gibt jedoch keinen Anhaltspunkt für eine chirale Auftrennung. Die in Schema 9 dargestellte Methode enthält eine chirale Auftrennung auf der Vorstufe des 3-Hydroxyesters, die sich durch das Fehlen der zentralen -(C=O)-CH₂- Einheit bezüglich der Stereochemie wesentlich von den 5-Hydroxy-3-ketoestern unterscheidet.

Der Erfindung liegt die Aufgabe zugrunde, ein Verfahren zur Herstellung optisch aktiver 5-Hydroxy-3-ketoester bereit zu stellen, durch das die beiden enantiomeren 5-Hydroxy-3-ketoester jeweils in einem Enantiomerenüberschuss von mindestens 95 % erhalten werden.

### Beschreibung der Zeichnung

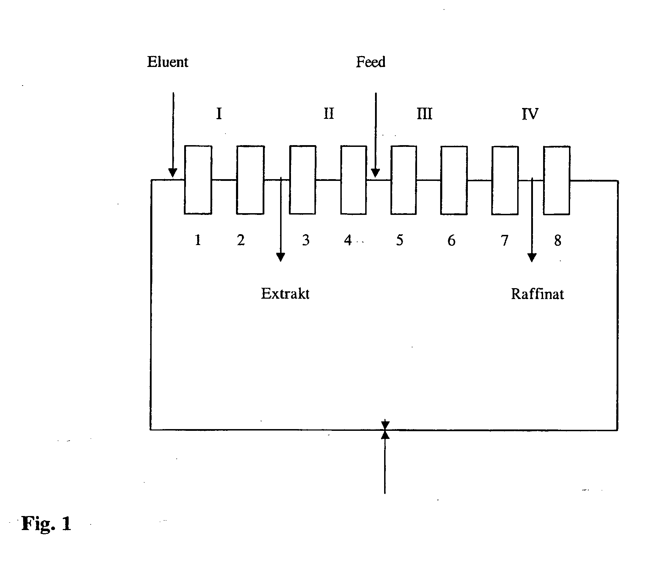

Figur 1 zeigt den schematischen Aufbau eines SMB (Simulated Moving Bed) Systems, welches vorteilhaft zur Durchführung des erfindungsgemäßen Verfahrens eingesetzt werden kann.

### Detaillierte Beschreibung der Erfindung

Die vorliegende Erfindung betrifft ein Verfahren zur Herstellung eines optisch aktiven 5-Hydroxy-3-ketoesters der Formel **A1** bzw. A2 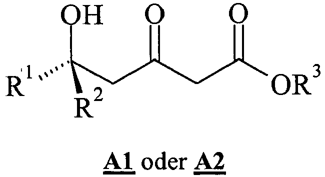 oder eines seiner Tautomeren,
worin R¹ 2-Phenylethyl und R² Propyl oder R¹ Propyl und R² 2-Phenylethyl bedeuten und R³ einen Rest ausgewählt aus der Gruppe bestehend aus tert.-Butyl und Ethyl darstellt, durch Auftrennen eines racemischen Gemischs eines 5-Hydroxy-3-ketoesters der Formel **A** 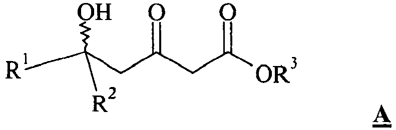 worin R¹, R² und R³ die oben angegebene Bedeutung haben,
in die beiden enantiomeren 5-Hydroxy-3-ketoester **A1** und **A2** mit präparativer Hochleistungsflüssigkeitschromatographie (HPLC) über ein chirales Trägermaterial, wobei man die beiden getrennten enantiomeren 5-Hydroxy-3-ketoester **A1** und **A2** jeweils in einem Enantiomerenüberschuss von mindestens 95 % erhält.

Ganz besonders bevorzugt stellen R¹ Phenylethyl, R² Propyl und R³ Ethyl oder tert.-Butyl dar. In der vorliegenden Erfindung sollen unter Alkylgruppen verzweigte und unverzweigte Alkylgruppen verstanden werden. Als Beispiele seien folgende Kohlenwasserstoffreste genannt: Propyl, 1-Methylethyl (Isopropyl) und 1,1-Dimethylethyl (tert.-Butyl).

Nachfolgend soll nun das erfindungsgemäße Verfahren im Einzelnen erläutert werden:

Nach dem Verfahren der Erfindung wird das racemische Gemisch **A** mit Hilfe der präparativen HPLC über ein chirales Trägermaterial nahezu quantitativ in die beiden enantiomeren 5-Hydroxy-3-ketoester **A1** und **A2** aufgetrennt.

Als HPLC-System (High Performance Liquid Chromatography) kann dabei eine bekannte Anlage eingesetzt werden. Derartige Anlagen sind aus mindestens einer Pumpe mit einem Elutionsmittelvorrat, einem Probenaufgabe-System, mindestens einer Trennsäule, einem Detektor, wie einem UV-Detektor, sowie einem Erfassungssystem aufgebaut. Heutzutage erfolgt die Steuerung derartiger Trennsysteme über Computeranlagen mit geeigneter Software, wodurch die Ventile geöffnet und geschlossen, die Pumpen gestartet und gestoppt und die Sensorenablesung vorgenommen wird. Es werden nicht nur die Parameter eingestellt und überwacht, sondern häufig auch die Auswertung hinsichtlich der Retentionszeiten (qualitative Bestimmung) und der Peak-Flächen (quantitative Bestimmung) mit entsprechender Software durchgeführt. Die HPLC-Technologie ist somit für Serienanalysen mit hohen Präzisionsanforderungen geeignet.

Die Säulen haben in der Regel Innendurchmesser zwischen etwa 1 mm und 3 m, vorzugsweise zwischen 50 mm und 1,5 m und eine Länge zwischen etwa 1 cm und 10 m, sind aus Stahl oder druckfestem Glas und mit einer stationären Phase gepackt.

Bei Verwendung eines HPLC-Systems nach dem erfindungsgemäßen Verfahren werden Säulen-Innendurchmesser im Bereich von etwa 20 cm bis 150 cm, vorzugsweise von 30 cm bis 120 cm, insbesondere von etwa 35 cm bis etwa 100 cm verwendet, wobei der Einsatz eines chiralen Trägermaterials als stationärer Phase von wesentlicher Bedeutung ist. Das erfindungsgemäß verwendete chirale Trägermaterial basiert auf einem Polysaccharid, das derart chemisch modifiziert wird, dass es optisch aktiv ist. Ein Beispiel hierfür ist, an das Polysaccharid-Grundgerüst ein oder mehrere optisch aktive Reste chemisch zu binden. Besonders bevorzugt wird das Polysaccharid ausgewählt aus Dextrin, Cyclodextrin, Stärke, Amylose und Cellulose.

Vorzugsweise verwendete chirale Trägermaterialien sind somit Tris(3,5-dimethyl-phenylcarbamat)-amylose, Tris[(S)-α-methylbenzylcarbamat]-amylose, Tris(3,5-dimethyl-phenylcarbamat)-cellulose, Tris(4-methylbenzoat)-cellulose, Cellulosetriacetat, Cellulosetribenzoat, Tris(phenylcarbamat)-cellulose, Tris(4-chlorphenylcarbamat)-cellulose, Cellulosetrizinnamat und Cellulosetribenzoat, von denen Tris(3,5-dimethylphenylcarbamat)-amylose und Tris(3,5-dimethylphenylcarbamat)-cellulose ganz besonders bevorzugt sind.

Das in der vorliegenden Erfindung verwendete chirale Trägermaterial ermöglicht es, das Racemat nahezu quantitativ in die beiden Enantiomeren aufzutrennen, wobei die chiralen Produkte mit hoher Enantiomerenreinheit über mindestens 95 % e.e. (enantiomer excess, Enantiomerüberschuß), vorzugsweise 96 bis 100 % e.e., insbesondere 98,2 bis 99,9 % e.e. erhalten werden. Daneben zeichnet sich das Trägermaterial durch seine lange Einsatzdauer bei gleichbleibend hoher Qualität aus, da es beispielsweise über Monate in der HPLC-Anlage zur Trennung verwendet werden kann und auch der Einsatz in einer kontinuierlich arbeitenden Anlage ohne weiteres möglich ist.

Als mobile Phase (Elutionsmittel oder Laufmittel) wird ein Lösemittel oder ein Lösemittelgemisch eingesetzt. Erfindungsgemäß sind als mobile Phase polare oder unpolare Lösemittel verwendbar. Geeignete organische Lösemittel umfassen beispielsweise Acetonitril, Propionitril, Butyronitril, Methylenchlorid, Chloroform, Hexan, iso-Hexan, Heptan, Methylacetat, Ethylacetat, n-Butylacetat, tert-Butylmethylether, iso-Propanol, Tetrahydrofuran, Dioxan, Methanol, Ethanol, Butanol, Toluol, Methylenchlorid, Chloroform, Methylacetat, Ethylacetat, n-Butylacetat und Wasser sowie Gemische hiervon, wobei sich in einem isokratischen System, d.h. einer Anlage in der nur ein Elutionsmittel eingesetzt wird, insbesondere Ethanol, Methanol, Butanol und iso-Hexan als vorteilhaft erwiesen haben. Besonders bevorzugt werden Gemische aus mindestens zwei Lösemitteln eingesetzt, wobei vorzugsweise ein polares mit einem unpolaren Lösemittel gemischt wird. Beispiele hierfür sind iso- und/oder n-Hexan/Ethanol, iso- und/oder n-Hexan/Methanol und iso- und/oder n-Heptan/Butanol. Das Verhältnis des polaren zum unpolaren Lösemittel hängt von der Polarität des zu trennenden Substrates und des Säulenmaterials ab und liegt in der Regel in einem Bereich von etwa 1:100 (v/v) bis etwa 100:1 (v/v), bevorzugt etwa 10:90 (v/v) bis etwa 90:10 (v/v).

Insbesondere bevorzugt ist der Einsatz von Tris(3,5-dimethylphenylcarbamat)-amylose mit dem Eluent Methanol, und von Tris(3,5-dimethylphenylcarbamat)-cellulose mit den Eluenten iso-Hexan/Ethanol oder iso-Hexan/Butanol.

Die weiteren Parameter des HPLC-Systems, wie zum Beispiel die Fließraten, Aufgabenmengen, Temperatur, Druck etc. hängen vom jeweils zu trennenden Racemat ab; diese können vom Durchschnittsfachmann anhand weniger orientierender Versuche ohne weiteres ermittelt und für jeden Einzelfall eingestellt und optimiert werden.

Das chromatographische Verfahren der Erfindung kann diskontinuierlich oder kontinuierlich geführt werden, wobei letztere Variante aus wirtschaftlichen Gründen bevorzugt ist. Es kann auch eine modifizierte Variante des HPLC-Systems zum Einsatz kommen, wie zum Beispiel die Technologie der SMB-Chromatographie (Simulated Moving Bed Chromatography). Hierbei handelt es sich um ein dem Fachmann bekanntes Konzept, wonach ein kontinuierlicher Gegenstrom zwischen einer mobilen und einer stationären Phase simuliert wird, und hierdurch bei relativ kurzer Laufzeit eine effiziente Trennung erfolgen kann. Dabei werden mehrere nacheinander geschaltete Säulen verwendet, die relativ kurze Längen aufweisen, mit verhältnismäßig kleinen Teilchen gepackt sind und kurze Umschaltzeiten zwischen den einzelnen Säulen haben. Der Zulauf wird kontinuierlich zugeführt und in zwei Produktströme aufgesplittet, mit denen die Trennung durchgeführt wird. Die prinzipiellen Einzelheiten derartiger Systeme sind dem Fachmann bekannt und bedürfen keiner weiteren Erläuterung.

Nur beispielhaft soll ein möglicher Aufbau eines SMB-Systems nachfolgend anhand der Figur 1 beschrieben werden:

Das SMB-System gliedert sich in vier Zonen (I bis IV), wobei 8 Säulen (Nr. 1 bis 8) verwendet werden, von denen immer zwei eine Zone definieren. Zone I stellt den SMB-Abschnitt dar, der zwischen der Zugabe von Eluent (Lösemittel, mobile Phase) und dem Extraktauslaß liegt. Die Zone II schließt sich an den Extraktauslaß an und endet beim Feedzulauf (Zugabe des zu trennenden Materials). Zone III ist zwischen dem Feedzulauf und dem Raffinatauslaß angeordnet und Zone IV schließt sich an den Raffinatauslaß an und reicht bis zum Eluent-Zulauf. Somit fließt das Eluat von Zone I bis Zone IV. Das Raffinat stellt das gewünschte Enantiomer, das Extrakt das andere Enantiomer dar.

Zweckmäßigerweise ist die Fließrate in Zone I ausreichend hoch, damit die mobile Phase in Zone II übertritt, wohingegen die Fließrate in Zone IV niedrig eingestellt wird, damit Raffinat entnommen werden kann.

Je nach dem Trennproblem modifiziert man die Anzahl der Durchläufe (Zykluszahl), die Fließrate in den vier Zonen des SMB-Systems (ml/min), die Einlaß und Auslaß-Fließrate (jeweils Eluent, Extrakt, Feed und Raffinat), insgesamt resultierend in einer durchschnittlichen Fließrate des Systems (ml/min), die Konzentration des zu trennenden Gemischs (Feed in g/l), die Durchlaufzeit pro Zyklus (min), den maximalen Druck während der Trennung (bar), die Menge und Art der stationären und mobilen Phase. Es ist dem Fachmann ohne weiteres möglich, die genannten Parameter einzustellen, um eine möglichst hohe Enantiomerenreinheit zu erzielen.

Ohne hierauf beschränkt zu sein, ist es erfindungsgemäß besonders vorteilhaft, die Bedingungen bei einem SMB-System in den folgenden Bereichen einzustellen:

| | |
|---|---|
| Anzahl der Säulen: | etwa 2 bis 10; |
| Säulen-Innendurchmesser: | etwa 0,1 bis 300 cm; |
| Säulen-Länge: | etwa 1 cm bis 10.000 cm; |
| chirale stationäre Phase: | chirales Trägermaterial mit einer Teilchengröße von etwa 3 bis 50 µm; |
| mobile Phase: | Lösemittel oder Lösemittelgemisch; |
| durchschnittliche Fließrate: | etwa 0,1 bis 10.000 ml/min; |
| UV-Detektion: | bei etwa 200 bis 300 nm; |
| Temperatur: | etwa 20 bis 60 °C; |
| gemessener Druck: | etwa 10 bis 50 bar. |

Die Produktivität der Anlage ergibt sich dann beispielsweise in g Enantiomer / Tag /kg chiraler stationärer Phase.

Auch mit einer derart aufgebauten SMB-Anlage ist es möglich, im erfindungsgemäßen Verfahrensschritt (2) eine Trennung des racemischen Gemischs **A** in die beiden Enantiomeren **A1** und **A2** jeweils mit einer Enantiomerenreinheit für das gewünschte Enantiomer von mindestens 98 % e.e., bevorzugt mindestens 99 % e.e. , insbesondere über 99,5% e.e. zu erhalten.

Das erfindungsgemäße Verfahren ermöglicht ohne weiteres den einfachen Zugang zu bislang nur schwer zugänglichen Enantiomeren, wobei dies auch in großtechnischem Maßstab mit ausgezeichneter Kosten/Nutzen-Relation wirtschaftlich möglich wird.

Das erfindungsgemäße Verfahren erlaubt es, das gewünschte Produkt nicht nur in hohen Ausbeuten, sondern auch mit sehr hoher Enantiomerenreinheit bereitzustellen, wobei keine komplizierte stereoselektive Synthese durchgeführt werden muß, sondern über das in einfacher Weise erhältliche racemische Gemisch vorgegangen werden kann. Es sind keine zusätzlichen Reinigungsschritte erforderlich, die Produkte können so wie sie anfallen direkt weiterverarbeitet werden. Es kann eine Standard-HPLC-Anlage verwendet werden, wobei die chromatgraphische Trennung kontinuierlich oder diskontinuierlich durchgeführt werden kann. Es können auch modifizierte Verfahren, wie die SMB-Chromatographie zum Einsatz kommen, wodurch eine noch bessere Optimierung der chromatographischen Trennung resultiert. Ausgehend vom racemischen Gemisch kann überraschenderweise eine Trennung in die beiden Enantiomeren mit einer Enantiomerenreinheit des gewünschten Enantiomeren über etwa 99,5 % e.e. erreicht werden, wobei dies nicht nur im Labormaßstab, sondern auch auf großindustriellen Anlagen in gewünschtem Umfang funktioniert.

Hierdurch wird der Zugang zu einer Substanzklasse wichtiger Arzneimittel möglich, den nicht-peptidischen HIV-Protease-Inhibitoren, wie Tipranavir, so dass die technische Lehre der Erfindung eine wertvolle Bereicherung für den Pharmaziesektor bedeutet.

Nachfolgend wird die Erfindung anhand von Beispielen, welche die erfindungsgemäße Lehre nicht beschränken sollen, im Einzelnen beschrieben. Dem Fachmann sind im Rahmen der erfindungsgemäßen Offenbarung weitere Ausführungsbeispiele offensichtlich.

### Referenz-Beispiel 1:

Die Herstellung des racemischen Gemischs von 5-Hydroxy-5-(2-phenylethyl)-3-oxooctansäureethylester (**1**) wurde folgendermaßen durchgeführt:

Ein Gemisch aus 260 ml (2,5 mol) Diethylamin und 500 ml Tetrahydrofuran wurde unter Stickstoff-Begasung bei Raumtemperatur vorgelegt. Es wurde auf -30 °C Innentemperatur abgekühlt. Bei dieser Temperatur wurden 1.000 ml (2,5 mol) n-Butyllithium in n-Hexan zugetropft. Man rührte 15 min nach und tropfte 158 ml (1,25 mol) Acetessigester zu. Nach nochmaligem 10 min Nachrühren tropfte man ein Gemisch aus 150 g (0,85 mol) 1-Phenyl-3-hexanon und 100 ml THF hinzu. Bei -30 °C wurde noch 2 h nachgerührt. Dann wurde die Reaktionslösung auf 1300 ml gesättigte NH₄Cl-Lösung gegeben. Die wässerige Phase wurde abgetrennt und die organische Phase 1x mit 500 ml gesättigter NH₄Cl-Lösung und etwa 300 ml 2N HCl geschüttelt. Die wässerige Phase wurde abgetrennt und die organische Phase 1x mit 300 ml gesättigter NaHCO₃-Lösung geschüttelt. Die organische Phase wurde wieder abgetrennt und im Vakuum eingeengt.

Ausbeute: 296 g (> 100% d. Th.; Th.= 260,4 g), NMR: 80%ige Reinheit, MS: MH⁺ = 307, (M-H)⁻ = 305

### Referenz-Beispiel 2:

Die Herstellung des racemischen Gemischs von 5-Hydroxy-5-(2-phenylethyl)-3-oxooctansäure-tert.-butylester (**2**) wurde folgendermaßen durchgeführt:

Ein Gemisch aus 260 ml (2,5 mol) Diethylamin, 150 ml (1,25 mol) 1,3-Dimethyltetrahydro-2(1H)-pyrimidon (DMPU) und 500 ml THF wurde unter Stickstoff-Begasung vorgelegt. Es wurde auf -15°C Innentemperatur abgekühlt und bei -15 bis -10 °C 1.000 ml (2,5 mol) n-Butyllithium in n-Hexan zugetropft. Danach wurden bei gleicher Temperatur in 1 Stunde 207 ml (1,25 mol) Acetessigsäure-tert.-butylester zugetropft. Anschließend tropfte man 150 ml (0,85 mol) einer Lösung aus 1-Phenyl-3-hexanon und 100 ml Tetrahydrofuran innerhalb einer Stunde zu und ließ dann 30 min nachrühren. Danach wurde die gekühlte Reaktionslösung auf 3.000 ml gesättigte Natriumbicarbonat-Lösung gegeben und anschließend die obere organische Phase abgetrennt. Die wässerige Lösung wurde verworfen und die organische Phase noch mit 3.000 ml 2N Salzsäure und im Anschluß daran mit 3.000 ml Wasser ausgeschüttelt. Dann wurde im Vakuum zur Trockene eingedampft.
NMR: 88 %-ige Reinheit

### Beispiel 3:

Das racemische Gemisch des 5-Hydroxy-3-ketoethylesters (**1**) von Referenz-Beispiel 1 wurde über ein SMB-System in die beiden enantiomeren Formen aufgetrennt. Das SMB-System enthielt 8 Säulen mit jeweils 1 cm Innendurchmesser und 10 cm Länge. Die Säulen wurden unter den folgenden Bedingungen gepackt:

| | |
|---|---|
| chirale stationäre Phase: | Chiralpak^{®}AD (Tris(3,5-dimethylphenylcarbamat)-amylose) |
| Lösemittel: | Isopropanol |
| Druck: | 300 bar |

Die Säulen wurden dann mit der mobilen Phase Methanol equilibriert und mit den nachfolgenden Bedingungen getestet:

| | |
|---|---|
| Probe: | 10 µl Trögers Base (lg/l) + TTBB (1,3,5,-tri-tert.-Butylbenzol 97%) (0,7 g/l) |
| Fließrate: | 4 ml/min |
| Druck: | 28 bar |
| UV-Detektion: | 250 nm |
| T°C: | 25°C |

Die durchschnittliche Fließrate im System war 279,3 ml/min; es wurden im System 35 bar Druck gemessen.

Die Einzelwerte waren: Fließrate_{Zone I} = 13,00 ml/min; Fließrate_{Extrakt} = 3,9 ml/min; Fließrate-_{Zone II} = 9,10 ml/min, Fließrate_{Feed} = 0,12 ml/min, Fließrate_{Zone III} = 9,22 ml/min, Fließrate_{Raffinat} = 1,14 ml/min, Fließratezone _{IV} = 8,08 ml/min, Feed = 100 g/l und ΔT (maximal gemessene Temperatur) = 80.

Das gewünschte Enantiomer wurde mit einer Reinheit von 99,40% e.e. erhalten (Raffinat). Das andere Enantiomer konnte mit einer Reinheit von 96,70% e.e. (Extrakt) isoliert werden.

### Beispiel 4:

Das racemische Gemisch des 5-Hydroxy-3-ketoethylesters (**1**) von Referenz-Beispiel 1 wurde über ein SMB-System in die beiden enantiomeren Formen aufgetrennt. Das SMB-System enthielt 8 Säulen mit jeweils 1 cm Innendurchmesser und 10 cm Länge. Die Säulen wurden unter den folgenden Bedingungen gepackt:

| | |
|---|---|
| chirale stationäre Phase: | Chiralcel^{®}OD (Tris(3,5-dimethylphenylcarbamat)-amylose) |
| Lösemittel: | Isopropanol |
| Druck: | 300 bar. |

Die Säulen wurden dann mit der mobilen Phase iso-Hexan/Isopropanol 90/10 equilibriert und mit nachfolgenden Bedingungen getestet:

| | |
|---|---|
| Probe: | 10 µl TSO (Trimeprazinsulfoxid) (2 g/l) + TTBB (1 g/l) |
| Fließrate: | 4 ml/min |
| Druck: | 21 bar |
| UV-Detektion: | 250 nm |
| T°C: | 25°C |

Alle Proben wurden dann mit den folgenden Bedingungen analysiert:

| | |
|---|---|
| mobile Phase: | iso-Hexan/Ethanol 98/2 |
| Träger: | Chiralcel^{®}OD (10 µm 250*4,6 mm) |
| Fließrate: | 0,5 ml/min |
| UV-Detektion: | 220 nm |
| Probeninjektion: | 10 µl |
| T°C | 25°C |
| Der gemessene Druck betrug 23 bar. | |

Die Einzelwerte waren: Fließrate_{Zone I} = 18,67 ml/min; Fließrate_{Extrakt} = 11 ml/min; Fließrate-_{Zone II} = 7,67 ml/min, Fließrate_{Feed} = 0,11 ml/min, Fließrate_{Zone III} = 7,78 ml/min, Fließrate_{Raffinat} = 1,65 ml/min, Fließrate_{Zone IV} = 6,13 ml/min, Feed = 100 g/l, ΔT = 203,5.

Um eine Überladung der Säule zu verhindern, wurde zunächst mit einer 50% niedrigeren Feed-Fließrate begonnen.

Das gewünschte Enantiomer wurde mit einer Reinheit von 99,64% e.e. erhalten (Raffinat). Das andere Enantiomer konnte mit einer Reinheit von 99,15% e.e. (Extrakt) isoliert werden.

### Beispiel 5:

Das racemische Gemisch des 5-Hydroxy-3-ketoethylesters (**1**) von Referenz-Beispiel 1 wurde über ein SMB-System in die beiden enantiomeren Formen aufgetrennt. Das SMB-System enthielt 8 Säulen mit jeweils 1 cm Innendurchmesser und 10 cm Länge. Die Säulen wurden unter den folgenden Bedingungen gepackt:

| | |
|---|---|
| chirale stationäre Phase: | Chiralcel^{®}OD |
| Lösemittel: | Isopropanol |
| Druck: | 300 bar. |

Die Säulen wurden dann wie in Beispiel 4 getestet:

| | |
|---|---|
| Probe: | 10 µl TSO (2 g/l) + TTBB (1 g/l) |
| Fließrate: | 4 ml/min |
| Druck: | 21 bar |
| UV-Detektion: | 250 nm |
| T°C: | 25°C |

Alle Proben wurden mit den folgenden Bedingungen analysiert:

| | |
|---|---|
| mobile Phase: | iso-Hexan/Butan-1-ol 90/10 |
| Träger: | Chiralcel^{®}OD (10 µm 250*4,6 mm) |
| Fließrate: | 1 ml/min |
| UV-Detektion: | 220 nm |
| Probeninjektion: | 10 µl |
| T°C: | 25°C |

Um eine Überladung der Säule zu verhindern wurde zunächst mit einer niedrigeren Feed-Fließrate begonnen. Bei den Anfangsbedingungen betrug der Druck etwa 15 bar. Die unterschiedlichen Fließraten wurden erhöht, um einen Druck von etwa 21 bar zu erhalten.

Die Einzelwerte waren: Fließrate_{Zone I} = 19 ml/min; Fließrate_{Extrakt} = 10,50 ml/min; Fließrate-_{Zone II} = 8,50 ml/min, Fließrate_{Feed} = 0,09 ml/min, Fließrate_{Zone III} = 8,59 ml/min, Fließrate_{Raffinat} = 2 ml/min, Fließrate_{Zone IV} = 6,59 ml/min, Feed = 100 g/l, ΔT = 121,2 und ΔP(maximal gemessener Druck) = 23.

Das gewünschte Enantiomer wurde mit einer Reinheit von 99,79% e.e. erhalten (Raffinat). Das andere Enantiomer konnte mit einer Reinheit von 99,39% e.e. (Extrakt) isoliert werden.

### Beispiel 6:

Das racemische Gemisch des 5-Hydroxy-3-keto-tert.-butylesters (**2**) von Referenz-Beispiel 2 wurde über ein SMB-System in die beiden enantiomeren Formen aufgetrennt. Das SMB-System enthielt 8 Säulen mit 4,8 mm Innendurchmesser und 10 cm Länge. Die Säulen wurden unter den folgenden Bedingungen gepackt:

| | |
|---|---|
| chirale stationäre Phase: | Chiralpak^{®}OD, 20 µm Teilchengröße |
| Lösemittel: | Isopropanol |
| Druck: | 300 bar |

Die Säulen wurden dann mit der mobilen Phase Isopropanol gespült und mit nachfolgenden Bedingungen getestet:

| | |
|---|---|
| Probe: | TSO (etwa 3g/l) + TTBB (0,5 g/l) in iso-Hexan/Isopropanol 90/10 - 10 µl eingespritzt |
| Fließrate: | 4 ml/min |
| UV-Detektion: | 250 nm |
| T°C: | 25°C |

Alle Proben wurden mit den folgenden Bedingungen analysiert:

| | |
|---|---|
| mobile Phase: | iso-Hexan/Ethanol 95/5 |
| Träger: | Chiralcel^{®}OD (10 µm 250*4,6 mm) |
| T°C: | 25°C |

Die Parameter auf einem SMB-System mit 8 Säulen eines Innendurchmessers von 4,8 mm, Länge 10 cm, 800 g chiraler stationärer Phase bei einer Feed-Konzentration von 90 g/l stellten sich wie folgt dar:

**Tabelle 1**

| **Parameter für einen 35 bar Druckabfall** | **erhaltene Werte** |
|---|---|
| Feed | 5,67 ml/min |
| Extrakt | 179,02 ml/min |
| Raffinat | 57,6 ml/min |
| Eluent | 230,86 ml/min |
| Umschaltzeit | 1,64 ml/min |
| Zone I | 464,03 ml/min |
| Zone II | 285,01 ml/min |
| Zone III | 290,77 ml/min |
| Zone IV | 233,17 ml/min |
| durchschnittliche Fließrate | 318,25 ml/min |
| Extrakt-Reinheit | 99,36% e.e. |
| Raffinat-Reinheit | 98,44% e.e. |
| Lösemittelverbrauch | 360,19 l/Tag |
| Produktivität | 493,21 g Enantiomer/kg stationäre Phase/Tag |

Das gewünschte Enantiomer wurde demnach mit einer Reinheit von 98,44% e.e. erhalten (Raffinat). Das andere Enantiomer konnte mit einer Reinheit von 99,36% e.e. (Extrakt) isoliert werden.

## Patentansprüche

1. Verfahren zur Herstellung eines optisch aktiven 5-Hydroxy-3-ketoesters der Formel **A1** bzw. **A2** 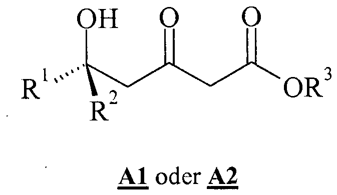 oder eines seiner Tautomeren,
worin
R¹ 2-Phenylethyl und R² Propyl oder R¹ Propyl und R² 2-Phenylethyl bedeuten und
R³ einen Rest, ausgewählt aus der Gruppe, bestehend aus tert.-Butyl und Ethyl darstellt, **dadurch gekennzeichnet, dass** man ein racemisches Gemischs eines 5-Hydroxy-3-ketoesters der Formel **A** 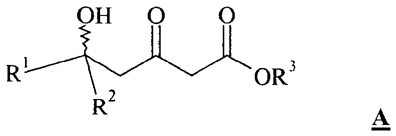 worin R¹, R² und R³ die oben angegebene Bedeutung haben,
in die beiden enantiomeren 5-Hydroxy-3-ketoester **A1** und **A2** mit präparativer Hochleistungsflüssigkeitschromatographie (HPLC) über ein chirales Trägermaterial auftrennt, wobei man die beiden getrennten enantiomeren 5-Hydroxy-3-ketoester **A1** und **A2** jeweils in einem Enantiomerenüberschuss von mindestens 95 % erhält.

2. Verfahren nach Anspruch1, **dadurch gekennzeichnet, dass** R¹ 2-Phenylethyl, R² Propyl und R³ Ethyl oder tert.-Butyl bedeutet.

3. Verfahren nach einem der Ansprüche 1 bis 2, **dadurch gekennzeichnet, dass** als chirales Trägermaterial ein chemisch modifiziertes Polysaccharid verwendet wird.

4. Verfahren nach Anspruch 3, **dadurch gekennzeichnet, dass** das chemisch modifizierte Polysaccharid ein Polysaccharid darstellt, das einen oder mehrere optisch aktive Reste chemisch gebunden enthält.

5. Verfahren nach Anspruch 3 oder 4, **dadurch gekennzeichnet, dass** das Polysaccharid ausgewählt wird aus der Gruppe, bestehend aus Dextrin, Cyclodextrin, Stärke, Amylose und Cellulose.

6. Verfahren nach mindestens einem der Ansprüche 3 bis 5, **dadurch gekennzeichnet, dass** das Trägermaterial ausgewählt wird aus Tris(3,5-dimethylphenylcarbamat)-amylose, Tris[(S)-α-methylbenzylcarbamat]-amylose, Tris(3,5-dimethylphenylcarbamat)-cellulose, Tris(4-methylbenzoat)-cellulose, Cellulosetriacetat, Cellulosetribenzoat, Tris(phenylcarbamat)-cellulose, Tris(4-chlorphenylcarbamat)-cellulose, Cellulosetrizinnamat und Cellulosetribenzoat.

7. Verfahren nach mindestens einem der Ansprüche 3 bis 6, **dadurch gekennzeichnet, dass** als Trägermaterial Tris(3,5-dimethylphenylcarbamat)-amylose oder Tris(3,5-dimethylphenylcarbamat)-cellulose eingesetzt wird.

8. Verfahren nach einem der Ansprüche 1 bis 7, **dadurch gekennzeichnet, dass** die präparative HPLC in Form der SMB (simulated moving bed) Chromatographie eingesetzt wird.

## Claims

1. Process for preparing an optically active 5-hydroxy-3-ketoester of formula **A1** or **A2** 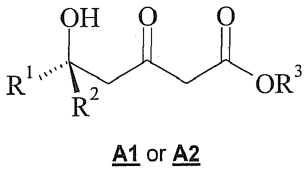 or one of the tautomers thereof,
wherein R¹ denotes 2-phenylethyl and R² denotes propyl or R¹ denotes propyl and R² denotes 2-phenylethyl, and
R³ denotes a group selected from among tert.-butyl or ethyl,
**characterised in that** a racemic mixture of a 5-hydroxy-3-ketoester of formula **A** 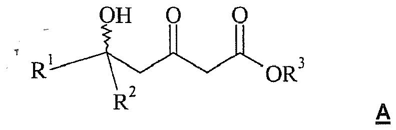 wherein R¹, R² and R³ are as hereinbefore defined,
is resolved into the two enantiomeric 5-hydroxy-3-ketoesters **A1** and **A2** by preparative high performance liquid chromatography (HPLC) over a chiral carrier material,
the two separate enantiomeric 5-hydroxy-3-ketoesters **A1** and **A2** each being obtained in an enantiomer excess of at least 95%.

2. Process according to claim 1, **characterised in that** R¹ denotes 2-phenylethyl, R² denotes propyl and R³ denotes ethyl or tert.-butyl.

3. Process according to one of claims 1 to 2, **characterised in that** a chemically modified polysaccharide is used as the chiral carrier material.

4. Process according to claim 3, **characterised in that** the chemically modified polysaccharide is a polysaccharide which contains one or more chemically-bound optically-active groups.

5. Process according to claim 3 or 4, **characterised in that** the polysaccharide is selected from among dextrin, cyclodextrin, starch, amylose and cellulose.

6. Process according to at least one of claims 3 to 5, **characterised in that** the carrier material is selected from tris(3,5-dimethylphenylcarbamate)-amylose, tris[(S)-α-methylbenzylcarbamate]-amylose, tris(3,5-dimethylphenylcarbamate)-cellulose, tris(4-methylbenzoate)-cellulose, cellulosetriacetate, cellulosetribenzoate, tris(phenylcarbamate)-cellulose, tris(4-chlorophenylcarbamate)-cellulose, cellulosetricinnamate and cellulosetribenzoate.

7. Process according to at least one of claims 3 to 6, **characterised in that** tris(3,5-dimethylphenylcarbamate)-amylose or tris(3,5-dimethylphenylcarbamate)-cellulose is used as the carrier material.

8. Process according to one of claims 1 to 7, charcaterised in that the preparative HPLC is used in the form of SMB (Simulated Moving Bed) chromatography.

## Revendications

1. Procédé de production d'un 5-hydroxy-3-cétoester optiquement actif de formule A1 ou A2 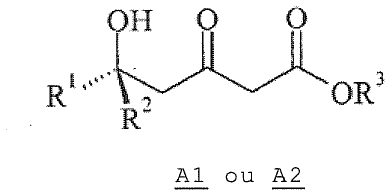 ou l'un de ses tautomères
dans laquelle
R¹ représente un groupe 2-phényléthyle et R² représente un groupe propyle ou R¹ représente un groupe propyle et R² représente un groupe 2-phényléthyle, et
R³ représente un radical choisi dans le groupe consistant en tert.-butyle et éthyle, **caractérisé en ce que** l'on sépare un mélange racémique d'un 5-hydroxy-3-cétoester de formule A 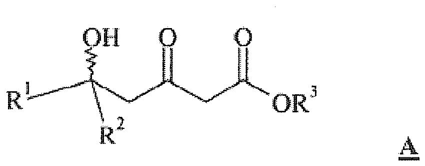 dans laquelle R¹, R² et R³ ont la signification indiquée ci-dessus,
en les deux 5-hydroxy-3-cétoesters énantiomères A1 et A2, par chromatographie préparative en phase liquide haute performance (HPLC) sur un matériau de support chiral, ce qui donne les deux énantiomères 5-hydroxy-3-cétoesters A1 et A2 séparés, respectivement, en un excès énantiomérique d'au moins 95 %.

2. Procédé selon la revendication 1, **caractérisé en ce que** R¹ représente un groupe 2-phényléthyle, R² représente un groupe propyle et R³ représente un groupe éthyle ou tert.-butyle.

3. Procédé selon l'une des revendications 1 à 2, **caractérisé en ce que** l'on utilise comme matériau de support chiral un polysaccharide modifié chimiquement.

4. Procédé selon la revendication 3, **caractérisé en ce que** le polysaccharide modifié chimiquement est un polysaccharide qui contient un ou plusieurs radicaux optiquement actifs liés chimiquement.

5. Procédé selon la revendication 3 ou 4, **caractérisé en ce que** le polysaccharide est choisi dans le groupe consistant en dextrine, cyclodextrine, amidon, amylose et cellulose.

6. Procédé selon au moins l'une des revendications 3 à 5, **caractérisé en ce que** le matériau de support est choisi parmi le tris(3,5-diméthylphénylcarbamate)-amylose, le tris[(S)-α-méthylbenzylcarbamate]-amylose, la tris(3,5-diméthylphénylcarbamate)-cellulose, la tris(4-méthylbenzoate)-cellulose, le triacétate de cellulose, le tribenzoate de cellulose, la tris(-phénylcarbamate)-cellulose, la tris(4-chloro-phénylcarbamate)-cellulose, le tricinnamate de cellulose et le tribenzoate de cellulose.

7. Procédé selon au moins l'une des revendications 3 à 6, **caractérisé en ce que** l'on utilise comme matériau de support, le tris(3,5-diméthylphénylcarbamate)-amylose ou la tris(3,5-diméthylphénylcarbamate)-cellulose.

8. Procédé selon l'une des revendications 1 à 7, **caractérisé en ce que** la HPLC préparative est utilisée sous la forme de chromatographie SMB (simulated moving bed / à lit mobile simulé).
